# EUROPEAN PATENT APPLICATION

(11) **EP 4 184 170 A1**
(43) Date of publication of application: **24.05.2023**
(21) Application number: 21849182.7
(22) Date of filing: 14.07.2021
(51) Int. Cl.: G01N 33/86, G01N 27/00

(54) **DEVICE FOR DETECTING BLOOD COAGULATION CHARACTERISTICS AND APPLICATION OF DEVICE**

(30) Priority: 31.07.2020 CN 202010759776
(71) Applicant: Micropoint Biotechnologies, Co., Ltd., Shenzhen, Guangdong 518133 (CN)
(72) Inventor: GAO, Yunfei, Shenzhen, Guangdong 518133 (CN); HUANG, Du, Shenzhen, Guangdong 518133 (CN); LIU, Feng, Shenzhen, Guangdong 518133 (CN)
(74) Representative: Canzler & Bergmeier Patentanwälte Partnerschaft mbB
(86) International application number: PCT/CN2021/106174
(87) International publication number: WO 2022/022280

(57) **Abstract**

A device 301 for determining blood coagulation characteristics. The device comprises: a blood sample receiving and pre-treating unit 201, comprising a blood pre-treating member 203, a sampling hole 212, and a conveying channel 210 connected to a reaction unit 204, the blood pre-treating member 203 being used for removing or counteracting an anticoagulation effect of an artificially added anticoagulant existing in a blood sample; the reaction unit 204, in which blood generates a coagulation process; a blood coagulation characteristic analysis and calculation unit 316 which is capable of detecting an electrical signal passing through the blood sample within a measurement duration to obtain a plurality of measurement results of a measurement function representing time; and a result presentation unit 320. Also provided is a method for detecting the blood coagulation characteristics of the blood sample by using the device.

## Description

### CROSS-REFERENCE TO RELATED APPLICATIONS

This application claims priority to and the benefit of Chinese Patent Application Ser. No. 202010759776.7 entitled "APPARATUS FOR TESTING BLOOD COAGULATION INDICATORS AND APPLICATIONS THEREOF" filed on July 31, 2020, the contents of which are incorporated herein by reference in its entirety.

### FIELD

The disclosure relates to a medical apparatus and method for testing blood coagulation indicators.

### BACKGROUND

In fields of medical assay and research, after collecting blood sample from a subject (e.g., collected from earlobe, fingertip, or vein), it is usually subjected to anticoagulation processing in order to prevent the blood sample from being clotted during storage or transfer. The anticoagulation processing includes, for example, artificially adding an anticoagulant such as sodium citrate. As such, the blood sample to test is likely anticoagulated blood with an artificially added anticoagulant.

Coagulation screening tests are usually required in clinical assay and routine screening, including assaying of Prothrombin Time (PT), Activated Partial Thromboplastin Time (APTT), Activated Clotting Time (ACT), Fibrinogen Content (FIB), and Thrombin Time (TT). Presently various assay meters have been developed for clinical laboratories and healthcare practitioners to test specific coagulation indicators.

However, the existed PT/APTT/FIB/TT assay systems and meters have certain drawbacks. As the test sample used by the clinical laboratory of a hospital is usually anticoagulated blood, e.g., sodium citrated blood, the anticoagulant included therein hinders occurrence of the sample's clotting process, such that the anticoagulated blood cannot be directly tested on the existing meters.

A meter and a corresponding method are still needed in the field, which enable directly using anticoagulated whole blood, e.g., sodium citrated whole blood, to quickly test the four coagulation indicators such as Prothrombin Time (PT), Activated Partial Thromboplastin Time (APTT), Fibrinogen Content (FIB), and Thrombin Time (TT).

### SUMMARY

The disclosure provides an apparatus for testing coagulation indicators of a blood sample, comprising:
a blood sample receiving and preprocessing unit comprising a blood preprocessing part, a sample loading hole, and a delivery channel connected to a reacting unit;
   and
the reacting unit in which blood undergoes a clotting process.
wherein the blood enters the sample loading hole after passing through the blood preprocessing part of the blood receiving and preprocessing unit, and then is delivered to the reacting unit through the delivery channel connected to the reacting unit in which the blood undergoes a clotting process.

In one aspect of the present disclosure, the apparatus for testing coagulation indicators of a blood sample further comprises:
a blood coagulation indicator analyzing and computing unit configured to obtain a measurement result related to a blood coagulation indicator based on a signal indicated by the blood during the blood clotting process in the reacting unit;
   and/or
a result displaying unit.

In one aspect of the present disclosure, the blood sample receiving and preprocessing unit and the reacting unit of the apparatus for testing coagulation indicators of a blood sample are standalone from other portions of the apparatus, e.g., they may be a test kit (e.g., in a form of chip) of an insertable meter (e.g., a meter comprising the blood coagulation indicator analyzing and computing unit and/or the result displaying unit).

In another aspect of the present disclosure, the blood sample receiving and preprocessing unit and the reacting unit of the apparatus for testing coagulation indicators of a blood sample are integrated with other portions of the apparatus (e.g., a meter comprising the blood coagulation indicator analyzing and computing unit and/or the result displaying unit) to form an all-in-one apparatus.

In one aspect of the present disclosure, the blood preprocessing part in the blood sample receiving and preprocessing unit is configured to remove or neutralize the anticoagulation effect of the anticoagulant (particularly artificially added) existed in the blood sample. In the present disclosure, the blood sample preprocessing refers to removing or neutralizing the anticoagulation effect of the anticoagulant (particularly of those being artificially added) existed in the blood sample without impacting inherent coagulation properties of the blood to be tested.

In one aspect of the present disclosure, the reacting unit comprises a module for applying a voltage difference to the blood sample and recording electrical signals. For example, the reacting unit comprises a power supply, a plurality of electrodes contacting the blood received in the reacting unit, and an A/D converter.

In one aspect of the present disclosure, the blood coagulation indicator analyzing and computing unit is configured to detect an electrical signal passing through the blood sample during the course of measurement, whereby Prothrombin Time (PT), International Normalized Standard (INR), Activated Partial Thromboplastin Time (APTT), Fibrinogen Content (FIB), and Thrombin Time (TT) of the sample are computed. In one aspect of the present disclosure, the blood coagulation indicator analyzing and computing unit comprises a processor, which may refer to any of a control logic circuit, a central processing unit, a microprocessor, or other appropriate types of processors.

In one aspect of the present disclosure, the blood preprocessing part may remove or neutralize the anticoagulation effect of sodium citrate, whereby the blood processing part may restore the coagulation properties of the sodium citrated blood sample.

In one embodiment of the present disclosure, the blood preprocessing part is prepared by a filter material. The filter material has a sheet porous body with many micro continuous pores connected from one surface to the other surface. In the present disclosure, the blood preprocessing part is a filter material part allowing for blood to pass through. In one embodiment of the present disclosure, the filter material has a aperture allowing particulate matter such as cells and follicles in the blood to pass through freely. By controlling the aperture as well as liquid passing distance and time of the filter material, the filter material would not produce or substantially would not produce physical adsorption to the cells, proteins or nucleic acids, various small molecules or ions in the blood when the blood passes through the filter material. Preferably, the material is biologically inert, which does not react with or inhibit the substances such as cells, proteins or nucleic acids in the blood. In one embodiment of the present disclosure, the filter material generally comprises a solid anticoagulant.

In one aspect of the present disclosure, the material of the blood preprocessing part is a polymer material, e.g., polyether sulfone (PES), polyacrylonitrile (PAN), polyvinylidene fluoride (PVDF), polystyrene (PS) and polyvinyl chloride (PVC), or a glass fiber. In another aspect of the present disclosure, the material of the blood preprocessing part is a hydrophilic material.

In one aspect of the present disclosure, the blood preprocessing part comprises a soluble calcium salt, e.g., calcium chloride. Soluble calcium salts can be used in the present disclosure include: calcium chloride, calcium gluconate, calcium dihydrogen phosphate, calcium nitrate, calcium hydrogen carbonate, and calcium hydrogen sulfate, etc. In one aspect of the present disclosure, the soluble calcium salt is present in the form of a solid in the blood preprocessing part. The soluble calcium salt in the blood preprocessing part is dissolved in the blood when the blood passes through, which provides free calcium ions for replenishing the calcium ions reduced or missed due to poorly dissociated soluble complexes formed with the citric acid radicals of the sodium citrate in the anticoagulated blood, thereby removing or neutralizing the anticoagulation effect of the sodium citrate.

In one aspect of the present disclosure, the blood preprocessing part is prepared by the polymer material contacting the soluble calcium salt solution.

In one aspect of the present disclosure, the blood preprocessing part is a filter block disposed over the sample loading hole or within the sample loading hole. The filter block generally has the same cross section as the sample loading hole, such that it may be disposed over the sample loading hole or within the sample loading hole. The filter block is configured such that the time and space for the blood sample to pass through the filter block allows the to-be-removed anticoagulant in the blood sample to sufficiently contact and react with the anticoagulant reversal agent contained in the filter block.

In another aspect of the present disclosure, the blood preprocessing part is a filter membrane covering the sample loading hole. One end of the filter membrane covers the sample loading hole, and the other end of the filter membrane is provided with a sample injection port. The blood added from the sample injection port moves transversely through the filter membrane to the sample loading hole. The filter membrane is configured such that the time and space for the blood sample to move transversely through the filter member from the sample injection port to the sample loading hole allow the to-be-removed anticoagulant in the blood sample to sufficiently contact and react with the anticoagulant reversal agent comprised in the filter membrane.

In one aspect of the present disclosure, the apparatus further comprises a processor. The processor may refer to any one of a control logic circuit, a central processing unit, a microprocessor, or other appropriate types of processors.

In one aspect of the present disclosure, the apparatus further comprises a computer readable medium. Information or computing algorithms associated with the blood coagulation indicators may be stored in the computer readable medium.

In one aspect of the present disclosure, the apparatus further comprises a displayer, which displays information associated with the blood coagulation indicators.

The present disclosure further provides a method for testing coagulation indicators of a blood sample, which is performed by the apparatus described above. In the present disclosure, the method comprises:
preprocessing the blood sample before delivery of the blood sample to the reacting unit which made the blood coagulate. In the present disclosure, the blood sample preprocessing refers to removing or neutralizing the anticoagulation effect of the artificially added coagulant existed in the blood sample, without impacting inherent coagulation properties of the to-be-tested blood itself.

In one aspect of the present disclosure, the blood preprocessing part may remove or neutralize the anticoagulation effect of sodium citrate, i.e., the blood preprocessing part may restore coagulation properties of the sodium citrated blood sample.

In one embodiment of the present disclosure, the blood preprocessing part is prepared by a filter material. In embodiments of the present disclosure, the filter material generally comprises a solid anticoagulant reversal agent.

In one embodiment of the present disclosure, the blood preprocessing part is a filter block disposed over the sample loading hole or within the sample loading hole. The filter block generally has the same cross section as the sample loading hole, such that the filter block may be disposed over the sample loading hole or within the sample loading hole. The filter block is configured such that the time and space for the blood sample to pass through the filter block allows the to-be-removed anticoagulant in the blood sample to sufficiently contact and react with the anticoagulant reversal agent.

In one aspect of the present disclosure, the blood preprocessing part is a filter membrane covering the sample loading hole. One end of the filter membrane covers the sample loading hole, and the other end of the filter membrane is provided with a sample injection port. The blood added from the sample injection port moves transversely through the filter membrane to the sample loading hole. The filter membrane is configured such that the time and space for the blood sample to move transversely through the filter member from the sample injection port to the sample loading hole allow the to-be-removed anticoagulant in the blood sample to sufficiently contact and react with the anticoagulant reversal agent.

In another aspect of the present disclosure, the method is configured to test at least one of the blood coagulation indicators set forth below:
Fibrinogen Content (FIB);
Thrombin Time (TT);
Prothrombin Time (PT);
Activated Partial Thromboplastin Time (APTT).

### BRIEF DESCRIPTION OF THE DRAWINGS

Specific embodiments of the present disclosure will be expounded with reference to the drawings below:

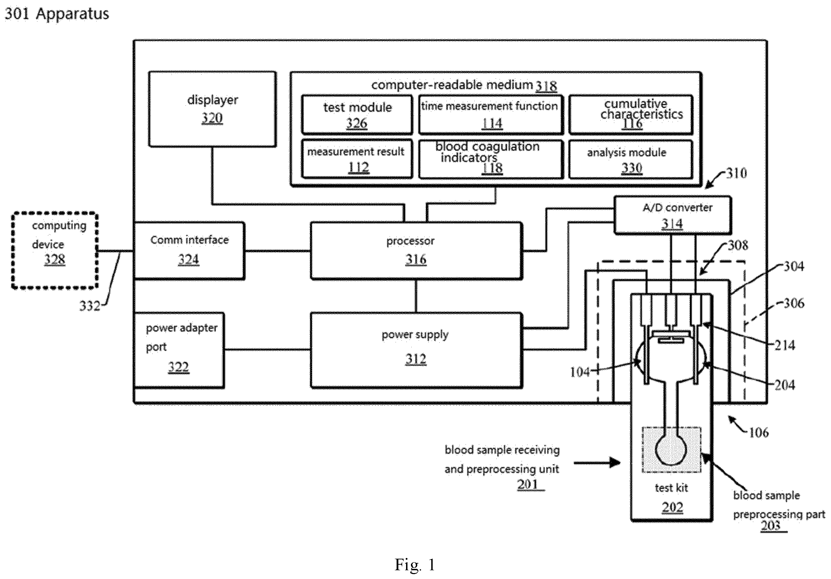

Fig. 1 is a structural schematic diagram of an exemplary apparatus 301 for testing blood coagulation indicators according to the present disclosure.

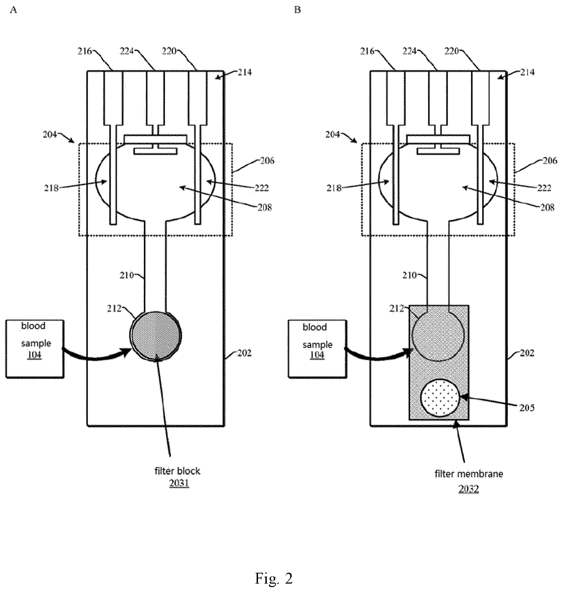

Fig. 2 is structural schematic diagram of an exemplary test kit 202 according to the present disclosure. Fig. 2A shows one example of the test kit provided by the present disclosure, wherein a blood preprocessing part is a filter column disposed over a sample loading hole or within the sample loading hole. Fig. 2B shows another example of the test kit provided by the present disclosure, wherein the blood preprocessing part is a filter membrane disposed over the sample loading hole.

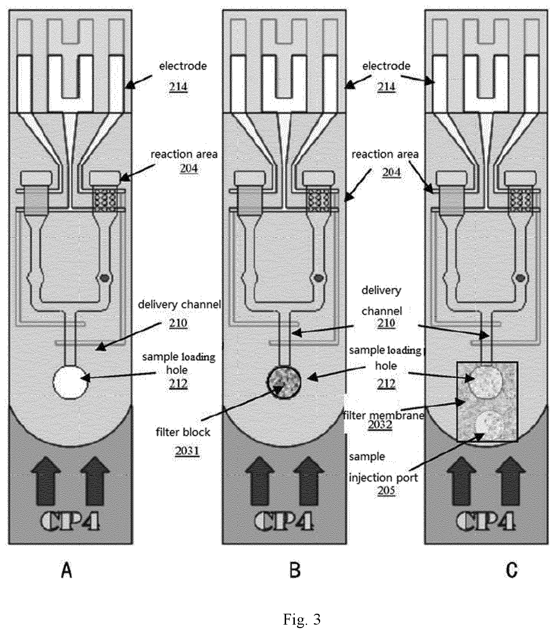

Fig. 3 illustrates structural schematic diagrams of the test kits adopted by apparatuses used in comparative testing, wherein Figs. 3A and 3B are test kits corresponding to the designs in Fig. 2A and Fig. 2B, respectively; and Fig. 3C is a test kit adopted by an apparatus in prior art, which does not have a blood preprocessing unit.

### DETAILED DESCRIPTION

For the sake of discussion, some exemplary embodiments are described in a context of an assay apparatus and system for testing blood coagulation indicators. However, it is apparent to those skilled in the art that the embodiments of the present disclosure are not limited to the specific examples provided herein, but may be extended to other types of equipment, apparatuses, and systems, as well as other types of assays.

The present disclosure provides an apparatus for testing blood coagulation indicators of a blood sample.

Fig. 1 is a structural schematic diagram of an exemplary apparatus for testing blood coagulation indicators according to the present disclosure.

As illustrated in Fig. 1, an apparatus 301 for testing blood coagulation indicators comprise a blood sample receiving and preprocessing unit 201, a reacting unit 204, a blood coagulation indicator analyzing and computing unit 316, and a result displaying unit 320.

In the exemplary apparatus for testing blood coagulation indicators illustrated in Fig. 1, the blood sample receiving and preprocessing unit 201 comprises a blood preprocessing part 203, a sample loading hole, and a delivery channel connected to the reacting unit. The blood sample receiving and preprocessing unit is configured to preprocess an original blood sample and then deliver the processed blood sample to the reacting unit 204. The blood sample receiving and preprocessing unit of the apparatus according to the present disclosure comprises a blood preprocessing part, a sample loading hole, and a delivery channel connected to the reacting unit.

In fields of medical assay and research, after collecting blood sample from a subject (e.g., collected from earlobe, fingertip, or vein), it is usually subjected to anticoagulation processing in order to prevent the blood sample from being clotted during storage or transfer. The anticoagulation processing includes, for example, artificially adding an anticoagulant such as sodium citrate. As such, the blood sample for test is likely anticoagulated blood with an artificially added anticoagulant. In the present disclosure, the blood sample preprocessing refers to removing or neutralizing the anticoagulation effect of the artificially added anticoagulant in the blood sample without impacting inherent coagulation properties of the to-be-tested blood itself.

The apparatus provided by the present disclosure is configured to test coagulation indicators of a subject's blood. Blood coagulation refers to a process of the blood to form blood clots, which generally comprises three phases: in the first phase, an injury or trauma to the wall of a blood vessel triggers platelet adhesion and activation; in the second phase, the activated platelets provide a surface for aggregation and activation of coagulation factors and compounds; in the third phase, the coagulation factors interact with each other to generate thrombin that converts fibrinogen into fibrin. Fibrin chains bind the aggregated platelets, facilitating reinforcement of the platelet-fibrin hemostatic plug. The second and third phases of blood coagulation may be referred to as coagulation cascade serving for interaction between each of the coagulation factors. The phases in which thrombin is formed are considered as main phases of blood clotting. Thrombin is a coagulation-effect enzyme, with multiple biologically important functions, e.g., activating platelets, converting fibrinogen into fibrin, and coagulation feedback and amplification. The coagulation cascade may be generalized into three pathways: intrinsic pathway, extrinsic pathway, and common pathway. The intrinsic pathway may be triggered by surface contract, while the extrinsic pathway may be triggered by tissue/cell defects. Either of the intrinsic and extrinsic pathways may lead to formation of thrombin. In the common pathway, the thrombin converts fibrinogen into fibrin, and then the fibrin may form a cross-linked fibrin mesh.

The blood coagulation indicators include, but are not limited to, Prothrombin Time (PT), Activated Partial Thromboplastin Time (APTT), Activated Clotting Time (ACT), Fibrinogen Content (FIB), and Thrombin Time (TT).

In the exemplary apparatus for testing blood coagulation indicators illustrated in Fig. 1, the reacting unit 204 is configured to cause the to-be-tested blood to undergo a biochemical or physiological reaction to thereby obtain information about the blood coagulation indicators. For example, the Chinese Patent No. CN103543191B discloses a method comprising: applying a voltage difference to a blood sample, and measuring a time-dependent electrical current response induced by coagulation reaction in the blood sample during the course of clotting, whereby blood coagulation indicators such as Prothrombin Time (PT), Activated Partial Thromboplastin Time (APTT), Activated Clotting Time (ACT), Fibrinogen Content (FIB), and Thrombin Time (TT) may be determined. A principle underlying the method is that: there exists an internal friction gradually increasing with electrical conductivity during the course of clotting, resulting in cumulation of electrical conduction energy losses.

In one embodiment of the present disclosure, all or part of components of the blood sample receiving and preprocessing unit and the reacting unit comprised in the apparatus for testing blood coagulation indicators are non-detachably provided in the apparatus 301.

In another embodiment of the present disclosure, all or part of components of the blood sample receiving and preprocessing unit and the reacting unit comprised in the apparatus for testing blood coagulation indicators are provided as a test kit detachably connected to the body of the apparatus 301. Preferably, the test kit is a single-use test kit, such that the components for receiving and accommodating the blood sample are also single-use, reducing risks of sample contamination and test result distortion.

In the exemplary apparatus for detecting blood coagulation indicators illustrated in Fig. 1, partial structures of the blood sample receiving and preprocessing unit and the reacting unit form a test kit 202, the test kit 202 being insertable into a test kit receiving slot 304 in the body of the apparatus 301.

Fig. 2 is a structural schematic diagram of an exemplary test kit 202 comprised in the apparatus of the present disclosure. When operating, the test kit 202 is inserted in the apparatus for testing blood coagulation indicators along a horizontal direction, i.e., Fig. 2 is a top-view plan of the test kit. Fig. 2A is an example of the test kit provided by the present disclosure, wherein the blood preprocessing part is a filter block 2031 disposed over the sample loading hole or within the sample loading hole. Fig. 2B is another example of the test kit provided by the present disclosure, wherein the blood preprocessing part is a filter membrane 2032 disposed over the sample loading hole.

As illustrated in Fig. 1 and Fig. 2, the blood sample receiving and preprocessing unit 201 comprises a blood preprocessing part 203. In the present disclosure, blood sample preprocessing refers to removing or neutralizing the anticoagulation effect of the artificially added anticoagulant in the blood sample without impacting inherent anticoagulation properties of the to-be-tested blood itself. Generally, the preprocessing may be implemented by contact between the blood sample and an anticoagulant reversal agent reacting with the to-be-removed artificially added anticoagulant. In an exemplary embodiment of the present disclosure, the to-be-processed blood sample is a sodium citrated blood sample, and the blood preprocessing part 203 is configured to remove or neutralize the anticoagulation effect of sodium citrate.

In one embodiment of the present disclosure, the blood preprocessing part is prepared with a filter material. The filter material has a sheet porous body with many micro continuous pores connected from one surface to the other surface. Examples of the porous body may include a membrane or block made of a polymer material (e.g., PES, PAN, PVDF, PS, PVC) or glass fiber. In one embodiment of the present disclosure, the filter material has an aperture allowing for particulate substances such as cells and follicles in the blood to pass through freely. By controlling the aperture of the filter material as well as the liquid passing distance and time, the filter material would not or substantially would not produce physical adsorption to the cells, proteins, nucleic acids, various small molecules or ions in the blood when the blood passes through the filter material. Preferably, the material is biologically inert, which does not react with or inhibit substances such as cells, proteins or nucleic acids in the blood. In some embodiments of the present disclosure, the filter material generally comprises a solid anticoagulant reversal agent.

In one embodiment of the present disclosure, the blood preprocessing part comprises a soluble calcium salt in the form of a solid. Soluble calcium salts applicable to the present disclosure include calcium chloride, calcium gluconate, calcium dihydrogen phosphate, calcium nitrate, calcium hydrogen carbonate, and calcium hydrogen sulfate, etc. In one embodiment of the present disclosure, the blood preprocessing part comprises calcium chloride. The soluble calcium salt may release free calcium ions in the blood, and the free calcium ions react with citric acid radicals of the sodium nitrate to form a poorly dissociated soluble complex, which reduces the sodium citrate in the anticoagulated blood, thereby removing or neutralizing the anticoagulation effect of the sodium citrate.

The soluble calcium salt may be bound onto the filter material forming the blood preprocessing part through adsorption. For example, by soaking the filter material in a solution containing the soluble calcium salt, followed by drying, a filter material comprising soluble calcium salt may be obtained, and then the resultant filter material may be processed into a desired shape.

In one embodiment of the present disclosure, the blood preprocessing part is a filter block disposed over a sample loading hole or within the sample loading hole, the filter block having the same cross section as the sample loading hole.

As illustrated in Fig. 2A, the test kit 202 comprises a sample loading hole 212 and a delivery channel 210 connected to the reacting unit. When operating, the test kit 202 is inserted into the apparatus for testing blood coagulation indicators along a horizontal direction, i.e., when operating, the filter column is perpendicular to the horizontal plane. A filter block 2031 is disposed over the sample loading hole 212, a sample injection port being provided at the top of the filter block. When the original blood sample is added dropwise into the sample injection port, the blood enters the filter block 2031, then flows through the filter column into the sample loading hole 212 under gravity and capillary action, and finally flows into a reaction area via the microfluidic delivery channel 210. The filter block, which generally has the same or similar cross section as the sample loading hole, may be disposed over the sample loading hole or within the sample loading hole. The filter block is configured such that the time and space for the blood sample to pass through the filter block allow the to-be-removed anticoagulant in the blood sample to sufficiently contact and react with the anticoagulant reversal agent.

In one embodiment of the present disclosure, the blood preprocessing part is a filter membrane disposed over the sample loading hole.

As illustrated in Fig. 2B, the test kit 202 comprises a sample loading hole 212 and a delivery channel 210 connected to the reacting unit, a filter membrane 2032 being disposed over the sample loading hole 212, one end of the filter membrane covering the sample loading hole 212, the other end of the filter membrane being provided with the sample injection port 205. The blood added from the sample injection port moves transversely through the filter membrane to the sample loading hole 212. The filter membrane is configured such that the time and space for the blood sample to move transversely through the filter member from the sample injection port to the sample loading hole allow the to-be-removed anticoagulant in the blood sample to sufficiently contact and react with the anticoagulant reversal agent.

As illustrated in Figs. 1 and 2, the test kit 202 further comprises a reaction area 204 represented by dashed block 206, the reaction area 204 comprising one or more reagents 208 and other substances selected for one or more test types. Generally, different tests (e.g., PT, APTT, ACT, FIB, or TT) have their own reagents 208 or other substances suitable for examining a specific indicator. As an example, the reagent 208 may be applied (e.g., printed, placed, etc.) and fixed in advance at the reaction area 204.

The test kit 202 may further comprise a plurality of electrodes 214 to form an electrochemical battery. For example, a first electrode 216 may be connected to a first portion 218 of the reaction area 204, and a second electrode 220 may be connected to a second portion 222 of the reaction area 204. And there may further be a third electrode 224 connected to the reaction area 204. In this example, the electrochemical battery at the reaction area 204 comprises three electrodes, e.g., a working electrode, a counter electrode, and a reference electrode. In another example, the counter electrode and the reference electrode may be combined, whereby the electrochemical battery has a two-electrode configuration.

Accordingly, it may be seen that when the blood from the sample injection port enters the reaction area 204, the blood sample 104 is located between a plurality of electrodes (e.g., the first electrode 216 and the second electrode 220). In addition, in an example where a reagent or an alternative substance is used for blood sample testing, the blood sample 104 may interact with the reagent 208 or the alternative substance in the reaction area 204. Moreover, although only a single electrochemical battery is illustrated in this example, a plurality of electrochemical batteries may also be comprised in other examples, whereby a plurality of reaction areas 204 are provided. For example, different reaction areas 204 may comprise different types of reagents 208 or substances suitable for different test types. Such reaction areas may be connected to a single-sample receiver 212 via passageways.

The blood sample and the reagent 208 react in the reaction area 204, which may further trigger a series of biochemical reactions leading to coagulation. In the reaction area 204, the blood sample 104 contacts the plurality of electrodes 214. When the test kit 202 is inserted into the apparatus 301, the electrodes 214 and the blood sample 104 in the reaction area 204 become an electrochemical portion of the integral circuit for testing the blood sample 104. For example, the apparatus may apply a voltage difference to the electrodes 214 for the purpose of detecting an electrical signal at the output terminal of the circuit within a certain period of time (i.e., within duration of the measurement), whereby a plurality of measurements of a function representing time (i.e., a time measurement function) are obtained.

The apparatus 301 for testing blood coagulation indicators according to the present disclosure further comprises a housing (not shown). The housing may isolate the inside of the meter from the atmosphere thereby prevent sample contamination and damages of the parts. A panel of the housing is usually made of a metal material. The housing may have a sample window that may be opened and closed, the sample window being configured for placing and removing the sample. The housing may additionally have an external device interface for connecting to an external device, a displayer for displaying an operating interface, and various manipulation switches, keys or buttons, etc.

As illustrated in Fig. 1, the apparatus 301 may further comprise a test kit receiving slot 304 and a heater 306. The heater 306 may ensure consistency of the temperature of the blood sample 104 during testing, thereby ensuring consistency in testing a plurality of samples.

The apparatus 301 may further comprise a plurality of electrical connections 308 for connecting the electrodes of the test kit 202 to the circuit 310 of the apparatus 302. For example, the electrodes 214 may be connected to a power supply 312 and an analog/digital (A/D) converter 314. The power supply 312 may apply a voltage difference to the blood sample 104 in the reaction area 204 of the test kit 202 during examination of the blood sample 104. The A/D converter 314 is configured to measure a resultant electrical signal for example at the output terminal. In some circumstances, the output terminal may correspond to one or more of the electrodes 214.

The apparatus 301 may further comprise one or more processors 316, one or more computer readable mediums 318, a displayer 320, a power adapter port 322, and one or more communication interfaces 324, e.g., an external data port or other appropriate communication interfaces. The processor 316 may refer to any one of a control logic circuit, a central processing unit, a microprocessor, or other appropriate processor types. In some examples, each processor 316 itself may comprise one or more processors or processing cores.

Dependent on configurations of the apparatus 302, the computer readable medium 318 may be an example of tangible non-transitory computer memory medium and may comprise volatile and non-volatile memories and/or removable and irremovable mediums implemented by any type of technology for storing information, e.g., computer readable instructions, data structures, program modules or other data. Such computer readable medium 318 may include, but not limited to, RAM, ROM, EEPROM, flash memory, or other computer readable medium technology, computer memory technology, or any other medium that may be used for storing information and may be accessed by the processor 316 directly or via another computing device. Correspondingly, the computer readable medium 318 may comprise a computer memory medium that may store and maintain instructions, modules or components executable by the processor 316.

The computer readable medium 318 may be used to store any number of functional components executable by the processor 316. In some embodiments, these functional components comprise instructions, codes or programs executable by the processor 316 and when being executed, implementing operational logic for executing the actions attributed to the apparatus 302. The functional components of the apparatus 302 maintained in the computer readable medium 318 may comprise a test module 326 which may be executed by the processor 316 to perform testing of blood samples, e.g., applying a voltage difference to the blood sample and obtaining a measurement result of the electrical signal. The functional components may further comprise an analysis module 330 which may be executed by the processor 316 to perform some functions described herein, for example determining the time measurement function 114, one or more corresponding cumulative characteristics 116, and/or the blood coagulation indicators 118. For example, the analysis module 330 may further determine the blood coagulation indicators at least partially based on the cumulative characteristics 116 and/or measurement results 112 or the time measurement function 114. In addition, the computer readable medium 318 may store data such as the measurement results 112, the time measurement function 114, the cumulative characteristics 116, and the blood coagulation indicators 118 or related information. Furthermore, based on the type of the apparatus 302, the computer readable medium 318 may optionally comprise other functional components and data, e.g., a user interface module, a communication module, a medical history, historical blood coagulation examination results, or other modules, applications, programs, drivers, data, etc.

The measurement results 112 obtained by the apparatus 301 with respect to the blood sample 104 may be stored in the computer readable medium 318 and be analyzed, for example, by the analysis module 330 executed by the processor 316. The analysis module 330 may determine quantitative values of the cumulative characteristics 116 of the measurement function 114. Before the measurement results 112 are obtained or before the apparatus 302 is manufactured in some cases, the cumulative characteristics 116 may be empirically derived or selected. For example, a criterion for selecting appropriate cumulative characteristics 116 is usually like: obtaining a good correlation with blood coagulation indicators 118 such that the cumulative characteristics 116 are usable in determining the blood coagulation indicators 118.

In some examples, the processing of determining the time measurement function 114, the cumulative characteristics 116, and/or the blood coagulation indicators 118 may be executed by the processor 316 by executing the analysis module 330 of the apparatus 301. In other examples, some or all of the processing may be executed by an external computing device 328. For example, the apparatus 302 may provide the measurement results 112 to the computing device 328, such that the computing device 328 may execute the analysis module 330 on the processor of the computing device 328 to determine the time measurement function 114, apply the cumulative characteristics 116, and/or determine the blood coagulation indicators 118. In this case, the test module 326 or another appropriate application or communication module on the apparatus 302 may communicate with the computing device 328 via the connection 332 to provide the measurement results 112 to the computing device 328. In some other examples, the computing device 328 may comprise a programming interface or API (Application Programming Interface) which may be used by the apparatus 302 to provide the measurement results 112. In some other examples, instead of providing measurement results 112, the apparatus 302 may provide the blood coagulation indicators 118 or information related to the blood coagulation indicators to the computing device 328.

The communication interface 324 may support wired and/or wireless connection 332 to access various networks, e.g., a cellular network, a radio communication, a Wi-Fi network, a short-range or near-field network (e.g., Bluetooth ^{®}), an infrared signal, a local area network, a wide area network, the Internet, and a direct cable connection, etc. As an example, the communication interface 324 may comprise a USB (Universal Serial Bus) port, an IEEE 802.3 port or other wired connections. In some other examples, the communication interface 324 may allow for the apparatus 301 to access a wireless communication network or device.

As illustrated in Fig. 1, the apparatus 301 further comprises a displayer 320. The displayer 320 may be a passive displayer, an emission displayer, or any other type of displayer. In some examples, the displayer 320 may be any appropriate type, e.g., a liquid crystal displayer, a plasma displayer, a LED displayer, an OLED displayer, etc. Additionally, in some examples, the displayer 320 may have a touch sensor associated therewith to enable touch input from a user. Furthermore, in some other examples, the apparatus 301 may not comprise a displayer.

### Embodiment 2 Testing of Blood Coagulation Indicators and Comparison of Results

### Test Items: Prothrombin Time (PT), Activated Partial Thromboplastin Time (APTT), Activated Clotting Time (ACT), Fibrinogen Content (FIB), and Thrombin Time (TT)

### 1. Meter

The testing uses the qSlabs^{®} Electrochemistry Analyzer (Model Q-1, YXZZ No. 20172401103) manufactured and sold by Micropoint Biotechnologies, Co., Ltd, which has been modified to obtain the apparatus provided by the present disclosure.

According to the user's manual of the Model Q-1 qSlabs^{®} Electrochemistry Analyzer, it may perform in vitro quantitative measurement of Prothrombin Time (PT), Activated Partial Thromboplastin Time (APTT), Activated Clotting Time (ACT), Fibrinogen Content (FIB), and Thrombin Time (TT) of human fresh anticoagulant-free venous whole blood or capillary whole blood. The structure of the analyzer comprises the apparatus 301 and various parts in the test kit 202 except the blood sample preprocessing part 203 as described in Embodiment 1 (as illustrated in Fig. 1 and Fig. 2), i.e., comprising the blood sample receiving and preprocessing unit 201, the reacting unit 204, the blood coagulation indicator analyzing and computing unit 316, and the result displaying unit 320, without the blood sample preprocessing part 203.

The qSlabs^{®} Electrochemistry Analyzer uses a single-use PT/APTT/FIB/TT quadruple test card, the top-view plan of which is illustrated in Fig. 3A; the meter comprises a structure of the test kit 202 schematically illustrated in Fig. 2 except the filter block 2031 or filter membrane 2032; i.e., comprising: the sample loading hole 212, the delivery channel 210, the reaction area 204, and the electrodes 214, etc.

When operating, the quadruple test card is inserted into the test card slot (equivalent to the test kit receiving slot 304 in Fig. 1) of the Model Q-1 qSlabs^{®} Electrochemistry Analyzer; After being added to the sample loading hole 212 , the blood sample enters the reaction area 204 via the delivery channel 210. When the analyzer is operating, a constant voltage is outputted to the electrodes 214. In the reaction area, the blood is triggered to undergo a series of biochemical reaction to start clotting. The electrochemistry analyzer monitors the blood clotting process; as current signal change occurs; the coagulation property analysis computer provided in the analyzer generates a coagulation curve reflecting changes of the electrical current and time according to a set program. With activation of the coagulation factors in the coagulation process and converts fibrinogen into fibrin, the curve changes and produces corresponding indicators (height, area, etc.). The coagulation indicators in the curve are analyzed to compute the Prothrombin Time (PT), International Normalized Ration (INR), Activated Partial Thromboplastin Time (APTT), Fibrinogen Content (FIB), and Thrombin Time (TT) of the sample, with the results being reported on the screen of the analyzer.

In this embodiment, the Model Q-1 qSlabs^{®} Electrochemistry Analyzer has been modified to additionally provide a blood preprocessing unit to thereby obtain the apparatus of the present disclosure. Specifically, the preprocessing unit plays a role of removing the anticoagulation effect of sodium citrate from the sodium citrated blood.

Figs. 3B and 3C are structural schematic diagrams showing test cards of two different designs adopted by the apparatus according to the present disclosure.

As illustrated in Fig. 3B, a filter block 2031 is disposed over the sample loading hole 212 of the test card. When operating, the test card is inserted into the qLabs^{®} Electrochemistry Analyzer along a horizontal direction, i.e., when operating, the filter column is perpendicular to the horizontal plane. The sodium citrated blood is added dropwise to the top of the filter column; then the blood enters the filter column, flows through the filter column into the sample loading hole under the capillary action, and finally enters the reaction area through a microfluidic delivery channel.

As illustrated in Fig. 3C, a filter membrane 2032 is disposed over the sample loading hole 212 of the test card, one end of the filter membrane covering the sample loading hole and the other end of the membrane being provided with the sample injection port 205. The sodium citrated blood is added dropwise in the sample injection port; after being adsorbed by the filter membrane, the blood moves transversely through the filter membrane into the sample loading hole, and then enters the reaction area through the microfluidic delivery channel.

### 2. Preparation of the Filter Column/ Filter Membrane

### Membrane Material for the Filter Column

| Name or Product No. | Source | Material | Aperture Size | Thickness | Hydrophilicity |
|---|---|---|---|---|---|
| PES precise filter liquid membrane | Shanghai Peihong New Materials Technology Co., Ltd. | Polyether Sulfone (PES) | About 1.2µm | About 0.1mm | Yes |

### Membrane Material for the Filter Membrane

| Name or Product No. | Source | Material | Aperture Size | Thickness | Hydrophilicity |
|---|---|---|---|---|---|
| Glass Fiber/8955 | Shanghai JN BIO | Glass fiber | 40±10s/4cm | 0.24±0.03mm | Yes |

### Binding Calcium Chloride to the Filter Column/ Filter Membrane

Calcium chloride (purity >96%, Sinopharm Chemical Reagent Co., Ltd.) is dissolved in deionized water to prepare a 0.05mol/L water solution; the membrane material set forth above are immersed in the calcium chloride solution for 10min, respectively; the soaked membrane materials are dried under 45 °C for 8h, ready for future use.

3. Blood Samples, which are collected from recruited volunteers by prinking fingertips; the blood samples collected from the same volunteer are prepared into a control sample and a test sample (sodium citrated blood), respectively.

### Control Samples: Original Fingertip Blood

Test Samples: 3.2% sodium citrate, sodium citrate: original blood = 1:9;

### 4. Testing

The (10) test samples are tested using the analyzer and settings illustrated in Fig. 3A. The results are all "No Coag," indicating no coagulation is detected in the blood samples.

The control samples and the test samples are tested by the analyzer and settings shown in Fig. 3B (5 samples, filter column: sample No. 1-5) or the analyzer and settings shown in Fig. 3C (5 samples, filter membrane: samples No.6-10), respectively.

The test results are set forth below:

| Sample Type | Fingertip Blood | | | | | Sodium Citrated Blood | | | | |
|---|---|---|---|---|---|---|---|---|---|---|
| Sample No. | PT | INR | APTT | FIB | TT | PT | INR | APTT | FIB | TT |
| 1 | 11.8 | 0.98 | 33.6 | 3.0 | 16.5 | 12.1 | 1.01 | 33.1 | 2.9 | 16.9 |
| 2 | 12.5 | 1.05 | 29.3 | 4.6 | 16.6 | 12.8 | 1.09 | 30.4 | 4.5 | 16.4 |
| 3 | 13.6 | 1.17 | 35.9 | 2.9 | 15.3 | 13.9 | 1.21 | 35.2 | 2.8 | 15.2 |
| 4 | 12.9 | 1.10 | 29.7 | 2.6 | 14.9 | 12 | 1.00 | 28.3 | 2.6 | 15.3 |
| 5 | 12.3 | 1.03 | 34.3 | 3.1 | 13.6 | 11.7 | 0.97 | 36.4 | 3.2 | 13.2 |
| 6 | 12.2 | 1.02 | 32.2 | 2.8 | 15.4 | 12.6 | 1.07 | 31.5 | 2.7 | 14.3 |
| 7 | 12.2 | 1.02 | 35.8 | 3.0 | 16.4 | 12.4 | 1.04 | 34.8 | 2.9 | 16.1 |
| 8 | 12.7 | 1.08 | 36.8 | 3.0 | 14.2 | 12.3 | 1.03 | 35.7 | 3.2 | 15 |
| 9 | 12.5 | 1.05 | 35.7 | 3.3 | 13.5 | 12.7 | 1.08 | 36.5 | 3.1 | 13.4 |
| 10 | 13.2 | 1.13 | 32.7 | 3.2 | 13.1 | 12.9 | 1.10 | 33.1 | 3.3 | 12.7 |

The deviation analysis is set forth below:

| Sample No. | INR | APTT | FIB | TT |
|---|---|---|---|---|
| 1 | 3.6% | -1.5% | -3.0% | 2.7% |
| 2 | 3.6% | 3.8% | -1.6% | -1.2% |
| 3 | 3.1% | -1.9% | -5.0% | -0.7% |
| 4 | -9.1% | -4.7% | -1.2% | 2.7% |
| 5 | -6.0% | 6.0% | 1.8% | -2.9% |
| 6 | 4.7% | -2.2% | -3.2% | -6.8% |
| 7 | 2.2% | -2.8% | -3.7% | -1.5% |
| 8 | -4.3% | -3.0% | 6.2% | 6.0% |
| 9 | 2.5% | 2.3% | -7.3% | -0.4% |
| 10 | -2.7% | 1.2% | 2.7% | -2.7% |

Result Analysis: Analysis of the testing results of the original fingertip blood and the sodium citrated blood unveils that after being subjected to the analyzer and settings of the present disclosure, the sodium citrated blood restores its clotting function, with the deviations between INR/APTT/FIB/TT measurement results of the fingertip blood and the anticoagulated blood being all within ± 10%.

## Claims

1. An apparatus for testing coagulation indicators of a blood sample, comprising:
a blood sample receiving and preprocessing unit comprising a blood preprocessing part, a sample loading hole, and a delivery channel connected to a reacting unit, wherein the blood preprocessing part is configured to remove or neutralize an anticoagulation effect of an anticoagulant existed in the blood sample; and
the reacting unit in which blood undergoes a clotting process.

2. The apparatus of claim 1, further comprising:
a blood coagulation indicator analyzing and computing unit configured to obtain a measurement result related to a blood coagulation indicator based on a signal indicated by the blood during the blood clotting process in the reacting unit, wherein the blood coagulation indicator analyzing and computing unit is configured to detect an electrical signal passing through the blood sample during the course of testing, whereby Prothrombin Time (PT), International Normalized Standard (INR), Activated Partial Thromboplastin Time (APTT), Fibrinogen Content (FIB), and Thrombin Time (TT) of the blood sample are computed; and/or
a result displaying unit.

3. The apparatus of claim 1 or 2, wherein the blood preprocessing part is prepared by a filter material allowing for the blood to pass through.

4. The apparatus of claim 3, wherein the material of the blood preprocessing part is a polymer material or a glass fiber.

5. The apparatus of claim 4, wherein the material of the blood preprocessing part is a polymer material selected from the group consisting of polyethersulfone (PES), polyacrylonitrile (PAN), polyvinylidene fluoride(PVDF), polystyrene(PS) and polyvinyl chloride (PVC) or a combination thereof.

6. The apparatus of claim 1, wherein the blood preprocessing part is configured to remove or neutralize the anticoagulation effect of sodium citrate.

7. The apparatus of claim 6, wherein the blood preprocessing part comprises soluble calcium salt.

8. The apparatus of claim 7, wherein the blood preprocessing part comprises calcium chloride.

9. The apparatus of claim 1, wherein the blood preprocessing part is a filter block disposed over the sample loading hole or within the sample loading hole.

10. The apparatus of claim 1, wherein the blood preprocessing part is a filter membrane covering the sample loading hole.

11. A method for testing blood coagulation indicators of a blood sample, which is implemented using the apparatus according to any one of claims 1 to 10, the method comprising:
preprocessing the blood sample before delivery of the blood sample to the reacting unit which made the blood coagulate.

12. The method of claim 11, wherein the method further comprises:
obtaining a measurement result related to a blood coagulation indicator based on a signal indicated by the blood during the clotting process in the reacting unit, wherein the blood coagulation indicator analyzing and computing unit is configurable to detect an electrical signal passing through the blood sample during the course of testing, whereby Prothrombin Time (PT), International Normalized Standard (INR), Activated Partial Thromboplastin Time (APTT), Fibrinogen Content (FIB), and Thrombin Time (TT) of the blood sample are computed; and/or
a result rendering unit.

13. The method of claim 11 or 12, wherein the blood preprocessing part is configurable to remove or neutralize the anticoagulation effect of sodium citrate, the blood preprocessing part is configured to restore coagulation properties of the sodium citrated blood sample.

14. The method of claim 13, wherein the blood preprocessing part is a filter block disposed over the sample loading hole or within the sample loading hole.

15. The method of claim 13, wherein the blood preprocessing part is a filter membrane covering the sample loading hole.

16. The method of claim 11, the method being adapted to test at least one of blood coagulation indicators set forth below:
Fibrinogen Content (FIB);
Thrombin Time (TT);
Prothrombin Time (PT);
Activated Partial Thromboplastin Time (APTT).
